# Europäisches Patentamt
# European Patent Office
## Office européen des brevets

(19)

(11) Veröffentlichungsnummer: **0 049 887**
**B1**

(12)

# EUROPÄISCHE PATENTSCHRIFT

(45) Veröffentlichungstag der Patentschrift:
29.05.85

(51) Int. Cl.⁴: **G 01 N 33/18** // C02F3/00

(21) Anmeldenummer: **81108173.6**

(22) Anmeldetag: **10.10.81**

(54) **Verfahren und Einrichtung zur Erfassung von biologisch abbaubaren und toxischen Inhaltsstoffen in wässrigen Lösungen, z.B. Abwasser.**

(30) Priorität: **10.10.80 DE 3038305**

(43) Veröffentlichungstag der Anmeldung:
**21.04.82 Patentblatt 82/16**

(45) Bekanntmachung des Hinweises auf die Patenterteilung:
**29.05.85 Patentblatt 85/22**

(84) Benannte Vertragsstaaten:
**AT BE CH DE FR GB IT LI NL SE**

(56) Entgegenhaltungen:
**DE - A - 2 415 771**
**DE - A - 2 728 071**
**DE - A - 2 935 120**
**DE - A - 2 951 707**
**US - A - 3 731 522**
**US - A - 3 740 320**
**US - A - 3 813 325**

**Die Akte enthält technische Angaben, die nach dem Eingang der Anmeldung eingereicht wurden und die nicht in dieser Patentschrift enthalten sind.**

(73) Patentinhaber: **Siepmann, Friedrich Wilhelm, Dipl.-Ing., Frankfurter Strasse 10, D-6100 Darmstadt (DE)**
Patentinhaber: **Teutscher, Michael, Dipl.-Ing., Heinrichstrasse 5, D-6111 Otzberg 4 (DE)**

(72) Erfinder: **Siepmann, Friedrich Wilhelm, Dipl.-Ing., Frankfurter Strasse 10, D-6100 Darmstadt (DE)**
Erfinder: **Teutscher, Michael, Dipl.-Ing., Heinrichstrasse 5, D-6111 Otzberg 4 (DE)**

(74) Vertreter: **Zinngrebe, Horst, Dr.rer.nat. et al, Saalbaustrasse 11, D-6100 Darmstadt (DE)**

**0 049 887**

## Beschreibung

Die Erfindung betrifft ein Verfahren zur Messung des Verschmutzungsgrades von wäßrigen Lösungen, z. B. Abwasser, durch biologisch abbaubare Inhaltsstoffe, bei dem der zu untersuchenden Lösung ein Teilstrom entnommen wird, der mit Sauerstoff durchlüftet und kontinuierlich durch ein biologisches Bad in einem Reaktionsbehälter hindurchgeleitet wird, hinter dem sein Restsauerstoffgehalt gemessen wird, wobei die Lebendmasse des Bades im wesentlichen auf konstantem Niveau gehalten wird, und eine Einrichtung nach dem Oberbegriff des Anspruchs 10.

Die Bestimmung des biologischen Sauerstoffbedarfs wird bisher im wesentlichen mit einem standardisierten Stichprobenverfahren durchgeführt, bei dem das Ergebnis nach fünf Tagen vorliegt (sog. $BSB_5$). Die Bedingungen hierfür sind im »Deutschen Einheitsverfahren H 4« festgelegt. Kontinuierlich arbeitende Geräte sind hierfür nicht bekannt.

In der US-PS 3 731 522 ist eine Vorrichtung zur Bestimmung des Sauerstoffverbrauchs von Abwasser beschrieben. In einer Einlaßleitung für einen Abwasserteilstrom zu einem geschlossenen Behälter sitzt eine erste Sauerstoffelektrode, und eine zweite Sauerstoffelektrode ist in der Auslaßleitung des Behälters angeordnet. Der Behälter wird von einem konstanten Abwasserstrom durchflossen, so daß sich durch Vergleich der an den Sauerstoffelektroden gemessenen Werte eine Aussage über den Sauerstoffverbrauch durch die in dem Abwasser enthaltenen Bakterien gewonnen werden kann.

In der DE-OS 2 415 771 wird ein Verfahren zur Bestimmung der Toxizität für die in der Sauerstoffzehrung zum Ausdruck kommenden Stoffwechselprozesse eines Abwassers vorgeschlagen, bei dem ein Differenzdruckmesser als einziges anzeigendes Gerät zum Einsatz kommt. Dabei wird die Tatsache ausgenutzt, daß in einem geschlossenen System das durch die Bakterienatmung entstehende Kohlendioxid mittels Kaliumhydroxid absorbiert wird, wodurch im System ein Unterdruck entsteht. Der Differenzdruckmesser wird gleichzeitig an geschlossene Systeme angeschlossen, die mit einer Wasserprobe unterschiedlicher Verdünnung gefüllt sind und deren eingetragene Wasservolumen im umgekehrten Verhältnis zur Verdünnung stehen.

In der US-PS 3 813 325 ist eine Vorrichtung mit einem von einem Abwasserteilstrom durchflossenen Behälter mit je einer Sauerstoffelektrode in der Eingangs- bzw. Ausgangsleitung beschrieben. Der Differenzwert der Sauerstoffmessungen kann wieder wie in der Vorrichtung gemäß US-PS 3 731 522 Aufschluß über die Sauerstoffzehrung in dem Behälter geben.

In der DE-OS 2 728 071 ist ein Verfahren zur Erkennung von bakterienschädigenden Inhaltsstoffen von Abwasser durch Messung der biologischen Aktivität von mit dem Abwasser versetztem Belebtschlamm beschrieben, bei dem die Sauerstoffzehrung eines Abwasser/Belebtschlammgemisches während eines vorbestimmten Zeitintervalls gemessen und die Messung mit einem neuen Abwasser/Belebtschlammgemisch wiederholt wird.

In der DE-OS 2 935 120 ist ein Verfahren zur Optimierung des Lufteintrags in ein Abwasser/Belebtschlammgemisch in Kläranlagen beschrieben, bei dem die Differenz der Sauerstoff-Partialdrücke der in ein Abwasser/Belebtschlammgemisch eingetragenen Luft und eines das Belebtschlammgemisch verlassenden Gases sowie die Lufteintragsrate ermittelt werden und die Lufteintragsrate auf einen Extremwert einer aus der Partialdruckdifferenz und der Lufteintragsrate gebildeten Funktion geregelt wird.

In der US-PS 3 740 320 wird ein Verfahren beschrieben, das generell durch die Bestimmung der Gaszehrung oder -freigabe in einem Abwasser die Messung der biologischen Aktivität des Abwassers gestattet. Dazu wird ein Behälter teilweise mit Abwasser gefüllt und zur Durchmischung und zum Ausgleich des Partialdruckes sowohl die Gasphase wie auch die Flüssigkeit ständig umgepumpt. In der Gasphase wird dabei der Verlauf des Sauerstoffgehaltes gemessen.

Schlußendlich wird in der DE-OS 2 951 707 ein Verfahren zur Bestimmung der Konzentration biologisch abbaubarer Stoffe in Abwässern nach der BSB-Methode beschrieben, bei welcher der Sauerstoffverbrauch in einer mit Belebtschlamm versetzten Abwasserprobe gemessen wird, wobei das Abwasser, der Belebtschlamm und die Sauerstoff enthaltende Meßluft einen Meßreaktor kontinuierlich durchströmen und die Sauerstoffkonzentrationsdifferenz der in den Meßreaktor einströmenden und der daraus austretenden Meßluft in einem Analysenteil gemessen wird.

Aus der DE-OS 2 514 609 ist ein Verfahren zum Messen der akuten Giftigkeit von wäßrigen Lösungen bekannt. Allerdings ist dabei z. B. für Abwasser keine Unterscheidung zwischen Verschmutzungsrückgang und leichten Vergiftungen möglich. Hieraus ergibt sich somit nur eine Positiv- oder Negativaussage.

Der Erfindung liegt die Aufgabe zugrunde, Verfahren und Einrichtungen zu schaffen, mit denen sich kontinuierliche Messungen der biologisch abbaubaren Verschmutzung durchführen lassen. Insbesondere sollen Hemmungen von Mikroorganismen durch Anwesenheit toxisch wirkender Substanzen in wäßrigen Flüssigkeiten, insbesondere in Abwässern oder in Fließgewässern, ermittelt werden können.

Zur Lösung dieser Aufgabe wurde als theoretische Grundlage das in vielen Experimenten bestätigte Modell von MICHAELIS und MENTEN herangezogen. Die Darstellung der biochemischen Reaktionsgeschwindigkeit in Abhängigkeit vom Nährstoffangebot stellt hierbei eine Hyperbel der Form

$$V = V_{max} \cdot \frac{L}{K_m + L} \tag{1}$$

Darin bedeuten

$V$     = Reaktionsgeschwindigkeit
$V_{max}$     = maximale Reaktionsgeschwindigkeit
$L$     = Nährstoffangebot (Konzentration)
$K_m$     = Michaelis-Konstante

Für kleine Nährstoffangebote ist die Michaelis-Gleichung in erster Näherung eine Grade der Form

$$V = V_{max} \cdot \frac{L}{K_m}. \qquad (2)$$

Praktisch bedeutet die Aussage der Gleichung (2): Bei niedrigen Substratkonzentrationen ist die Reaktionsgeschwindigkeit der Konzentration proportional, d. h., eine Nährstofferhöhung ruft in diesem Bereich eine lineare Sauerstoffzehrung hervor. Voraussetzung dafür ist allerdings, daß der Sauerstoffgehalt nicht unter 15% seines Sättigungswertes sinkt, damit er keine »Schrittmacherreaktion« hervorruft.

Die Michaelis-Gleichung gilt im strengen Sinne nur für eine konstante Enzymmenge bei variabler Substratkonzentration und bei ungehinderter Diffusion der Nährstoffe zum Enzym. Diese Bedingungen lassen sich, wie Versuche gezeigt haben, durch treibende Hohlkörper als Aufwuchsflächen der Organismen, konstant hohe Umwälzgeschwindigkeit im Reaktionsbehälter und damit gleiche Organismenaufwuchs- und -abspülraten sicherstellen.

Ausgehend hiervon besteht die Lösung der Aufgabe erfindungsgemäß in einer Abwandlung des eingangs genannten, aus der DE-OS 2 514 609 bekannten Verfahrens, die dadurch gekennzeichnet ist, daß der Teilstrom mit biologisch neutralem Wasser verdünnt wird und daß die Verdünnung des Teilstroms durch Messung des Sauerstoffgehaltes vor dem Reaktionsbehälter und Vergleich mit dem Meßergebnis des Restsauerstoffgehaltes derart geregelt wird, daß bei konstantem Volumenstrom durch den Reaktionsbehälter die Differenz der Meßwerte im wesentlichen bei einem vorgegebenen Wert konstant bleibt, wobei der Verdünnungsgrad zur Anzeige des Verschmutzungsgrades dient.

Der Sauerstoffgehalt des Teilstroms der zu untersuchenden Flüssigkeit wird also am Zu- und Ablauf des Reaktionsbehälters, in dem sich Aufwuchsflächen für Mikroorganismen befinden, gemessen und die Diferenz in einer geringen Schwankungsbreite konstant gehalten, indem bei kleiner werdender Sauerstoffdifferenz der zugesetzte Anteil an Verdünnungswasser verringert, bei größer werdender Differenz erhöht wird. Dadurch wird eine konstante Substratversorgung und eine konstante Sauerstoffzehrung der Mikroorganismen im Reaktionsbehälter sichergestellt. Bei gleichbleibender Aufwuchsfläche im Behälter stellt sich durch kontinuierlichen Abrieb und Abspülung auch eine konstante Organismenmenge ein. Besonders geeignet sind treibende Hohlkörper, die gegen Abrieb geschützte Aufwuchsflächen bieten.

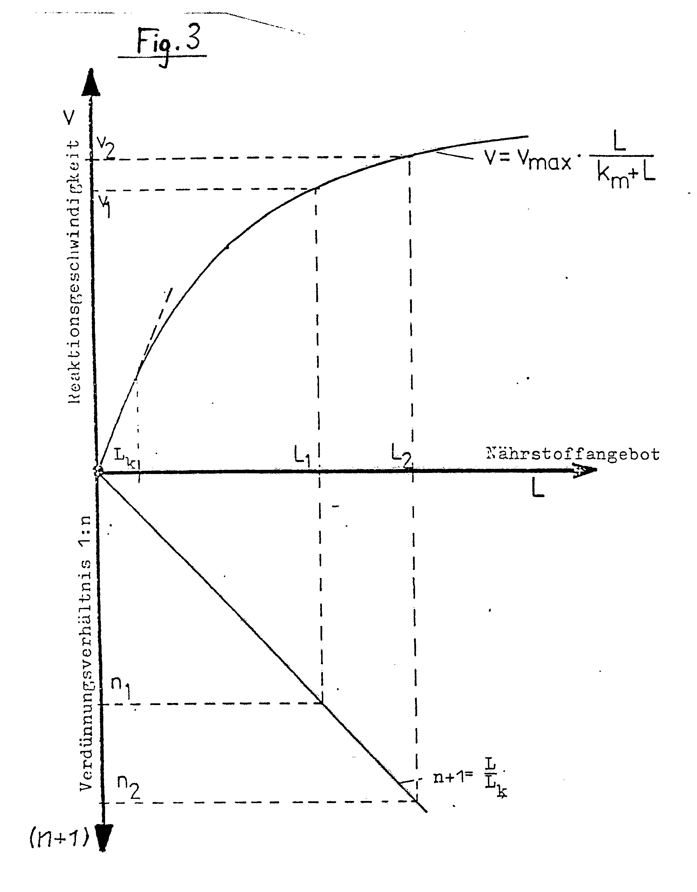

Bei der erfindungsgemäßen Ausführung soll die dem Reaktionsbehälter zugeführte Konzentration »$L_k$« (Fig. 3) aus dem Bereich der angenähert linearen Abhängigkeit der Reaktionsgeschwindigkeit der Lebendmasse vom Nährstoffangebot gewählt werden. Dies ist i. a. zu erwarten, wenn die Reaktionsgeschwindigkeit kleiner als die halbe maximale Reaktionsgeschwindigkeit ist ($v_k < v_{max}/2$). In diesem Bereich kann sowohl eine sehr spontane Reaktion der Lebendmasse auf Konzentrationsschwankungen erwartet, als auch eine lineare Abhängigkeit zu Grunde gelegt werden.

Bei zu erwartenden pH-Schwankungen der zu untersuchenden Flüssigkeit können hemmende Wirkungen auf die Mikroorganismen durch entsprechende Zudosierung einer biologisch neutralen Pufferlösung verhindert werden.

Durch das Aufbringen eines leichten Überdrucks in der Anlage kann sowohl eine bessere Sauerstofflöslichkeit erreicht als auch das Entstehen von Gasblasen in Anlagenteilen verhindert werden.

Bei erfindungsgemäß konstant gehaltener mikrobieller Aktivität im Reaktionsbehälter kann aus dem Verdünnungsverhältnis sowie aus der gemessenen $O_2$-Differenz und der Temperatur eine Aussage getroffen werden über die biologisch abbaubare Verschmutzung der zu untersuchenden Flüssigkeit, die durch Vergleichsmessungen und Eichen der Apparatur auch direkt als $BSB_5$-Wert ausgedrückt werden kann. Anwendungsbereiche dieses BSB-Meßverfahrens sind z. B. die Registrierung von biologisch abbaubaren Schmutzfrachten in Kläranlagen und die Steuerung solcher Anlagen nach dem aktuellen Sauerstoffbedarf.

Bei Anwesenheit toxischer Inhaltsstoffe in der zu untersuchenden Flüssigkeit tritt eine Hemmung der Mikroorganismen ein, deren Grad von der Konzentration der toxischen Stoffe abhängt.

Zur Bestimmung der Toxizität wird in Ausgestaltung des erfindungsgemäßen Verfahrens der zu untersuchenden Lösung ein zweiter Teilstrom entnommen und in gleicher Weise behandelt, wobei jedoch der Verdünnungsgrad des zweiten Teilstroms um einen konstanten Faktor m niedriger eingeregelt wird als der des ersten Teilstroms und die Differenz der Meßwerte vor und hinter dem zweiten Reaktionsbehälter mit den Meßwerten aus dem ersten Teilstrom verglichen und als Toxizitätsgrad angezeigt wird.

Bei parallelem Betrieb zweier Reaktionsbehälter, bei dem die Verdünnung im ersten Behälter nach dem beschriebenen Verfahren geregelt wird, dem zweiten Behälter aber immer ein um den Faktor m weniger verdünnter Teilstrom zugeleitet wird, so daß hier eine toxische Substanz auch immer in der m-fachen Konzentration vorliegt, können aus einer verminderten Sauerstoffzehrung im zweiten Behälter dann Rückschlüsse auf das Vorliegen und den Grad einer toxischen Wirkung gezogen werden, wenn der Verdünnungsgrad im ersten Behälter so eingestellt wird, daß die toxischen Inhaltsstoffe in diesem noch nicht hemmend wirken.

Bei der erfindungsgemäßen Toxizitätsmessung handelt es sich also um zwei parallel durchgeführte BSB-Messungen mit unterschiedlichem Verdünnungsgrad. Da die hemmende Wirkung toxischer Substanzen bei steigender Konzentration überproportional zunimmt, läßt sich der Toxizitätsgrad als Verhältnis der in beiden Teilströmen a, b gemessenen Aktivität $BSB_a/BSB_b$, bezogen auf den Verdünnungsgrad im ersten Behälter, ausdrücken.

Anwendungsbereich des Verfahrens ist z. B. sein Einsatz als Warnungssystem für biologische Anlageteile in Kläranlagen vor toxischen Abwasserstößen.

In Weiterbildung der Erfindung wird zur Bestimmung der toxischen Wirkung wäßriger Lösungen in Absolutwerten zunächst in den Reaktionsbehältern mit einer Nährlösung bekannter Zusammensetzung ohne jeglichen toxischen Einfluß eine »Standardbiologie« gezüchtet und aufrechterhalten. Dann wird die vergiftete wäßrige Lösung der »Standardbiologie« zugeführt und dabei die Aktivität der Organismen, für deren ungehemmte atmungsaktivität aufgrund vorangegangener Messungen sogenannte Nullpunktwerte vorliegen, registriert. Mit einer so geeichten diskontinuierlichen Messung lassen sich dann absolute Giftigkeiten, bezogen auf eine »Standardbiologie«, angeben.

Die Erfindung bezieht sich ferner auf Einrichtungen zum Messen der biologisch abbaubaren Verschmutzung in wäßrigen Flüssigkeiten, insbesondere in Abwässern und Fließgewässern, sowie zum Messen der hemmenden Wirkung toxischer Inhaltsstoffe auf Mikroorganismen.

Die Einrichtung zur Ausführung des Verfahrens mit einem Belüftungsgefäß und einem Reaktionsbehälter, durch die der aus der wäßrigen Lösung entnommene Teilstrom hindurchfließt, mit einer Sauerstoffelektrode in der Ablaufleitung aus dem Reaktionsbehälter sowie einer weiteren Sauerstoffelektrode in einer den Auslauf des Belüftungsgefäßes mit dem Zulauf des Reaktionsbehälters verbindenden Leitung kennzeichnet sich nach der Erfindung dadurch, daß auf der Einlaufseite des Belüftungsgefäßes die Zuleitung des Teilstroms mit einer Zuleitung für Verdünnungswasser zusammengeführt ist, daß in der Zuleitung für Verdünnungswasser eine an eine Steuerung angeschlossene Durchfluß-Regelvorrichtung liegt und die weitere Sauerstoffelektrode an die Steuerung angeschlossen ist, wobei die Steuerung einen Vergleicher und einen Stellsignalgeber für die Durchfluß-Regelvorrichtung enthält.

Die Durchfluß-Regelvorrichtung kann zweckmäßig in der Weise realisiert sein, daß in der Zuleitung für den Teilstrom und in der Zuleitung für Verdünnungswasser je eine Dosierpumpe angeordnet ist.

Die Aufwuchsflächen im Reaktionsbehälter bestehen dabei aus frei beweglichen Körpern mit gegen Abrieb geschützten Aufwuchsflächen. Körper und Behälterinhalt werden zweckmäßig durch eine Rühr- oder Umwälzvorrichtung ständig durchmischt. Vor der Einleitung in den Belüfter kann die zu untersuchende Flüssigkeit gegebenenfalls ein Sieb und ein Rührwerk zur weitgehenden Homogenisierung durchlaufen.

Mit den gemessenen $O_2$-Werten wird das Verhältnis der Zuflußmengen der zu untersuchenden Flüssigkeiten und des Verdünnungswassers über einen Prozeßrechner so gesteuert, daß sich bei konstanter Durchströmung des Reaktionsbehälters eine konstante $O_2$-Differenz einstellt und am Auslauf ein Mindestwert nicht unterschritten wird. Die Stellgröße wird zur Anzeige ausgewertet.

Ein zweiter gleichartiger Reaktionsbehälter, der in Reihe mit dem ersten Reaktionsbehälter geschaltet und mit einer vorgeschalteten und einer nachgeschalteten Sauerstoffelektrode ausgerüstet ist, kann zur Bestimmung der Abbaubarkeit der Verschmutzung des Teilstroms sowie alternativ zur Toxizitätsmessung benutzt werden.

Zur Bestimmung der Toxizität durch Reihenschaltung der BSB-Messung wird in Ausgestaltung des erfindungsgemäßen Verfahrens die zu untersuchende Lösung ein zweitesmal in gleicher Weise behandelt, wobei jedoch der Verdünnungsgrad im zweiten Reaktionsbehälter um einen konstanten Faktor m niedriger eingeregelt wird als der im ersten Reaktionsbehälter und die Diferenz der Meßwerte vor und hinter dem zweiten Reaktionsbehälter mit den Meßwerte aus dem ersten Reaktionsbehälter verglichen und als Toxizitätsgrad angezeigt wird.

Bei in Reihe geschaltetem Betrieb zweier Reaktionsbehälter, bei dem die Verdünnung im ersten Behälter nach dem beschriebenen Verfahren, allerdings über die Sauerstoffelektroden des nachgeschalteten Behälters, geregelt wird, dem zweiten Behälter aber immer ein um den Faktor m stärker verdünnter Teilstrom zugeleitet wird, so daß hier eine toxische Substanz auch immer in der m-fach geringeren Konzentration vorliegt, können aus einer verminderten Sauerstoffzehrung im ersten Behälter dann Rückschlüsse auf das Vorliegen und den Grad einer toxischen Wirkung gezogen werden, wenn der Verdünnungsgrad im nachgeschalteten Behälter so eingestellt wird, daß die toxischen Inhaltsstoffe in diesem noch nicht hemmend wirken.

Bei dieser Toxizitätsmessung handelt es sich also um zwei hintereinander durchgeführte BSB-Messungen mit unterschiedlichem Verdünnungsgrad. Da die hemmende Wirkung toxischer Substanzen bei steigender Konzentration überproportional zunimmt, läßt sich der Toxizitätsgrad als Verhältnis der in beiden Reaktionsbehältern gemessenen Aktivität, bezogen auf den Verdünnungsgrad im zweiten Behälter, ausdrücken.

Wenn die Mikroorganismen durch eine starke toxische Wirkung nachhaltig geschädigt werden, können die bewachsenen Schwebekörper aus dem Reaktionsbehälter entnommen und gegen einen neuen Satz mit unvergifteter Biozönose ausgetauscht werden.

Im folgenden wird die Erfindung anhand der Zeichnungen erläutert. Es zeigt

Fig. 1 eine Einrichtung zur Messung der BSB,

Fig. 2 eine Einrichtung zur gleichzeitigen Messung von BSB und Toxizität,

Fig. 3 Reaktionsdiagramme,

Fig. 4 eine schematische Darstellung einer weiteren Ausführungsform der Erfindung und

Fig. 5 eine schematische Darstellung einer weiteren Ausführungsform der Erfindung.

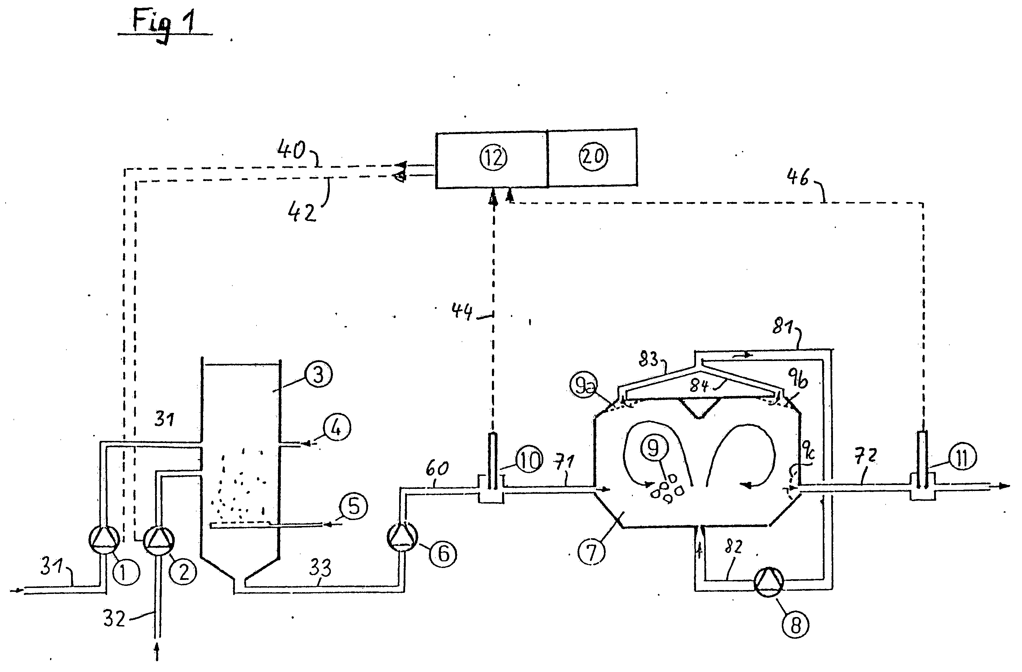

Gemäß Fig. 1 werden die zu untersuchende Flüssigkeit durch die Dosierpumpe 1 und das Verdünnungswasser durch die Dosierpumpe 2 der Zulaufseite eines Belüftungsgefäßes 3 zugeleitet, in welchem dem Gemisch eine Lösung zur pH-Pufferung über eine weitere Zuleitung 4 zugegeben wird. Durch Eintragen von Luft oder gasförmigem Sauerstoff mittels einer Zuleitung 5 wird die im Belüftungsgefäß 3 enthaltene Flüssigkeit mit Sauerstoff angereichert. Der vom Belüftungsgefäß 3 über eine Auslaufleitung 33 abgezogene Flüssigkeitsstrom wird durch eine Pumpe 6 in konstantem Strom einem Reaktionsbehälter 7 über eine Zulaufleitung 71 zugeführt. In der die Zulaufleitung 71 mit der Pumpe 6 verbindenden Leitung 60 ist eine erste Sauerstoffelektrode 10 eingeschaltet, die den Sauerstoffgehalt und die Temperatur der strömenden Flüssigkeit mißt und die gemessenen Werte einem in einer Steuerung enthaltenen Prozeßrechner 12 zuführt.

Der über die Zuleitung 71 in den nach außen geschlossenen Reaktionsbehälter 7 eintretende Flüssigkeitsstrom verläßt diesen durch eine am gegenüberliegenden Ende des Reaktionsbehälters 7 angeordnete Ablaufleitung 72, in der eine weitere Sauerstoffelektrode 11 angeordnet ist. Die Sauerstoffelektrode 11 mißt die Temperatur und den Sauerstoffgehalt des Auslaufstroms und leitet die gemessenen Werte in Form von Signalen dem Prozeßrechner 12 zu. Auf der Abstromseite der Sauerstoffelektrode 11 mündet die Ablaufleitung 72 in einen nicht dargestellten Ablaufbehälter.

Der Reaktionsbehälter 7 ist in eine Umwälzleitung 81, 82 geschaltet, durch die die im Reaktionsbehälter 7 enthaltene Flüssigkeit mittels einer Umwälzpumpe 8 fortwährend umgewälzt wird. Die an die Abstromseite der Umwälzpumpe 8 angeschlossene Umwälzleitung 82 mündet in den Boden des Reaktionsbehälters 7, und die Flüssigkeit aus dem Reaktionsbehälter 7 aufnehmende Umwälzleitung 81 ist über mehrere Zweigleitungen 83, 84 mit dem Deckel des Reaktionsbehälters 7 verbunden.

Im Reaktionsbehälter sind eine große Zahl von Schwebekörpern 9 mit Aufwuchsflächen für die Mikroorganismen enthalten, die durch die umgewälzte Flüssigkeit im Reaktionsbehälter 7 in beständiger Bewegung sind. Vor den in die Leitungen 83 und 84 sowie 72 mündenden Ausgangsöffnungen aus dem Reaktionsbehälter 7 sind Rückhaltesiebe 9a, 9b, 9c mit einer im Verhältnis zu den Schwebekörpern 9 kleinen Maschenweite angeordnet. Alternativ zum Umwälzkreislauf 81, 82 kann im Reaktionsbehälter 7 auch ein nicht dargestelltes, entweder vom Zustrom aus der Zuleitung 71 oder separat von außen angetriebenes Rührwerk vorgesehen sein.

Die Steuerung enthält ein vom Prozeßrechner 12 gesteuertes Anzeigegerät 20, das den vom Prozeßrechner 12 ermittelten BSB-Wert anzeigt oder ausdruckt. Aus dem Prozeßrechner 12 führt eine Steuerleitung 40 zu der Dosierpumpe 1 in der Zulaufleitung 31 für das Belüftungsgefäß 3 sowie eine Steuerleitung 42 zur Dosierpumpe 2 in der Zulaufleitung 32 für Verdünnungswasser. Die Steuerleitungen 44, 46 zwischen der ersten Sauerstoffelektrode 10 bzw. der zweiten Sauerstoffelektrode 11 und dem Prozeßrechner 12 übertragen die von den Sauerstoffelektroden ermittelten Meßwerte an den Prozeßrechner 12.

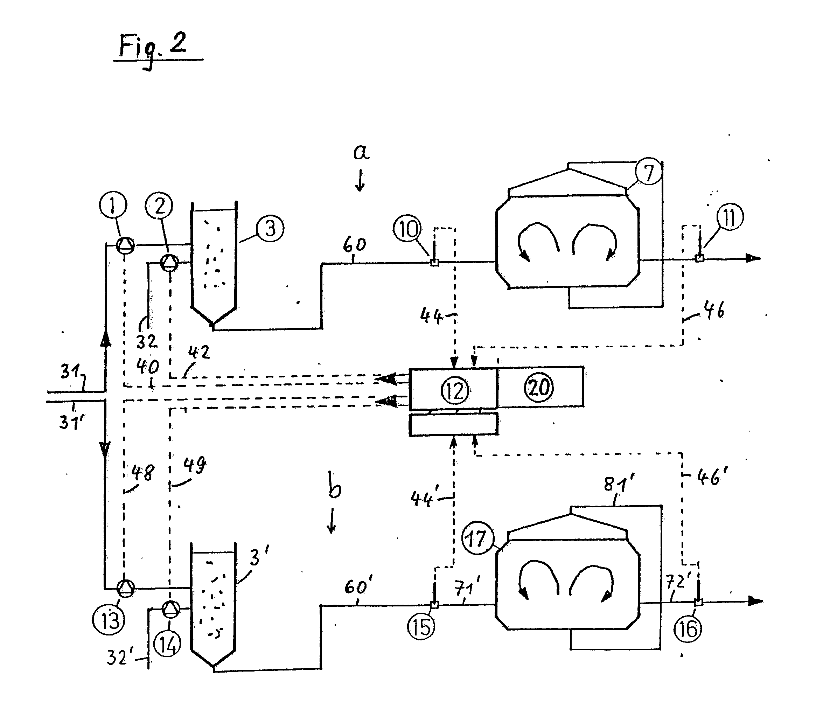

In Fig. 2 ist die Einrichtung nach Fig. 1 in der oberen Hälfte schematisch noch einmal dargestellt, und im unteren Teil ist eine weitere gleichartige Einrichtung zur Toxizitätsmessung schematisch erläutert. Diese weitere Einrichtung umfaßt grundsätzlich die gleichen Geräte und Bauteile, wie die vorstehend beschriebene Einrichtung und umfaßt daher eine weitere Zuleitung 31' zur Einlaufseite eines Belüftungsgefäßes 3' zur Zuführung von verschmutzter Lösung, wobei in der Zuleitung 31' eine weitere Dosierpumpe 13 angeordnet ist. An der Aufstromseite kann die Zuleitung 31' mit der Aufstromseite der Zuleitung 31 verbunden sein, so daß beide Zuleitungen 31 und 31' an einen nicht dargestellten Behälter mit verschmutzter, zu untersuchender Lösung angeschlossen sind. Auf der Einlaufseite des weiteren Belüftungsgefäßes 3' befindet sich eine Zulaufleitung 32' für Verdünnungswasser mit einer Dosierpumpe 14. In der Ablaufleitung 60' vom Belüftungsgefäß 3' ist eine weitere Sauerstoffelektrode 15 angeordnet, die den Sauerstoffgehalt und die Temperatur des Flüssigkeitsstroms über eine Steuerleitung 44' dem Prozeßrechner 12 über einen Vergleicher 13', der die Differenz der Meßwerte der Elektroden aufnimmt, zuführt. Die Sauerstoffelektrode 15 ist über eine Zulaufleitung 71' mit einem weiteren Reaktionsbehälter 17 gekoppelt, der dem Reaktionsbehälter 7 gleicht und ebenfalls in einen Umwälzkreislauf 81' geschaltet ist. Die Ablaufleitung 72' aus dem Reaktionsbehälter 17 weist eine weitere Sauerstoffelektrode 16 auf, die den gemessenen Sauerstoffgehalt und die Temperatur des Abstroms über eine Steuerleitung 46' dem Prozeßrechner 12 über den Vergleicher 13' zuführt. Vom Prozeßrechner 12 führt eine weitere Steuerleitung 48 zur Dosierpumpe 13 für einlaufende, verschmutzte Flüssigkeit sowie eine Steuerleitung 49 zur Dosierpumpe 14 für Verdünnungswasser. Der Prozeßrechner 12 beaufschlagt über die Steuerleitung 48, 49 die Dosierpumpen 13, 14 in der Weise, daß die Konzentration der dem Belüftungsgfäß 3' insgesamt zugeführten Flüssigkeit an verunreinigter Flüssigkeit immer das m-fache der entsprechenden Konzentration der dem Belüftungsgefäß 3 zugeführten Flüssigkeit ist. Mit den Meßwerten der Sauerstoffelektroden 15 und 16 im Vergleich zu den Meßwerten, die an den Sauerstoffelektroden 10, 11 gewonnen werden, kann eine eventuelle toxische Hemmung der Mikroorganismen nachgewiesen werden.

Die Einrichtung nach Fig. 2 dient auch als Toximeter zur diskontinuierlichen Messung mit Standardbiologie. Für Meßpausen, zur Züchtung und Regenerierung der Standardbiologie werden beide Anlagenteile mit einer standardisierten Nährlösung betrieben.

Aus Fig. 3 ist zu ersehen, daß durch die konstante geringe Nährstoffversorgung »$L_k$« die Organismen immer im idealisiert linearen Bereich arbeiten:

$$V = V_{max} \ \frac{L}{K_m} \triangleq n+1 = \frac{L}{L_k}.$$

Während $V_1$ und $V_2$ bei hohen Nährstoffkonzentrationen L und zu berücksichtigenden Meßungenauigkeiten nicht eindeutig unterscheidbar sind, bleiben n und L immer proportional zueinander.

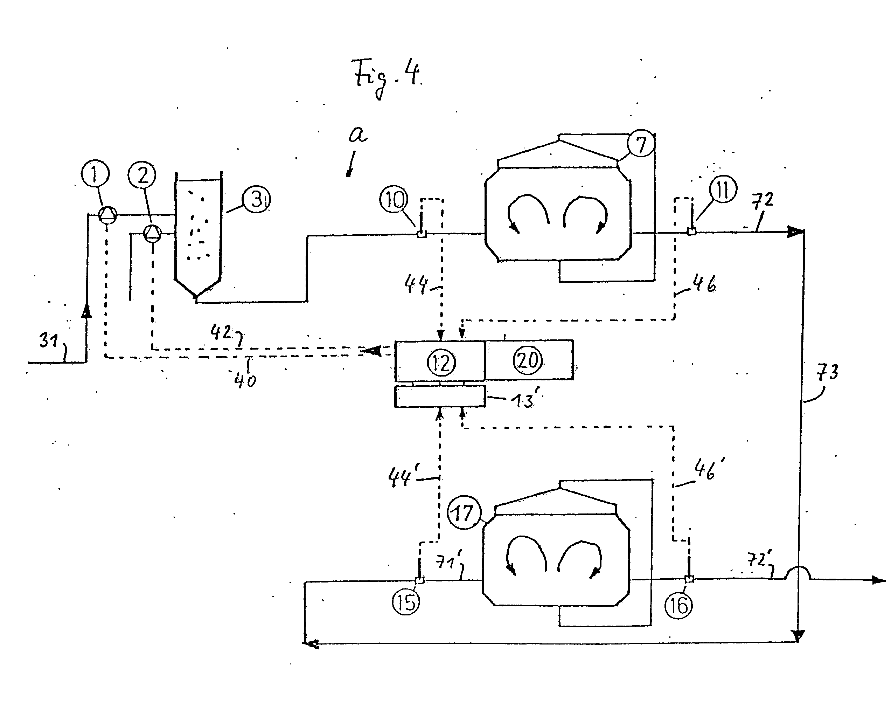

Bei der Einrichtung nach Fig. 4 ist an die Ablaufleitung 72 des Teilstroms a eine Üpbergangsleitung 73 angeschlossen, die mit der zweiten Sauerstoffelektrode 15 verbunden ist, welche über eine Zulaufleitung 71' mit dem zweiten Reaktionsbehälter 17 sowie über eine Steuerleitung 44' mit dem Vergleicher 13 der Steuerung verbunden ist. Die in die Ablaufleitung 72' des Reaktionsbehälters 17 eingeschaltete Sauerstoffelektrode 16 ist über die Steuerleitung 46' mit dem Vergleicher 13 gekoppelt. Diese Reihenschaltung der beiden Reaktionsbehälter 7 und 17 ermöglicht außer der BSB-Messung auch die Bestimmung der Abbaubarkeit der Verunreinigung im Teilstrom a. Ein BSB-Wert bestimmter Größe kann von leichter oder schwerer abbaubaren Stoffen herrühren. Bislang hat man diesem Umstand dadurch Rechnung getragen, daß man den $BSB_5$-Wert in Verbindung mit einem CSB (chemischer Sauerstoffbedarf) ermittelte. Bei dem mit der Einrichtung gemäß Fig. 4 ausführbaren Verfahren wird den Mikroorganismen im ersten Reaktionsbehälter eine konstante Verschmutzung im Teilstrom a zugeführt. Diese Verschmutzung wird, abhängig von der Zusammensetzung der Verschmutzung, bis auf einen Restwert im ersten Reaktionsbehälter abgebaut. Diese Restverschmutzung in dem die Übergangsleitung 73 durchströmenden Teilstrom ruft je nach ihrer Größe unterschiedliche Sauerstoffdifferenzen zwischen den Sauerstoffelektroden 15 und 16 beim Durchlauf durch den zweiten Reaktionsbehälter 17 hervor. Diese unterschiedlichen Sauerstoffdifferenzen stellen ein Kriterium zur Beurteilung der Abbaubarkeit der im Teilstrom a enthaltenen Verschmutzung dar.

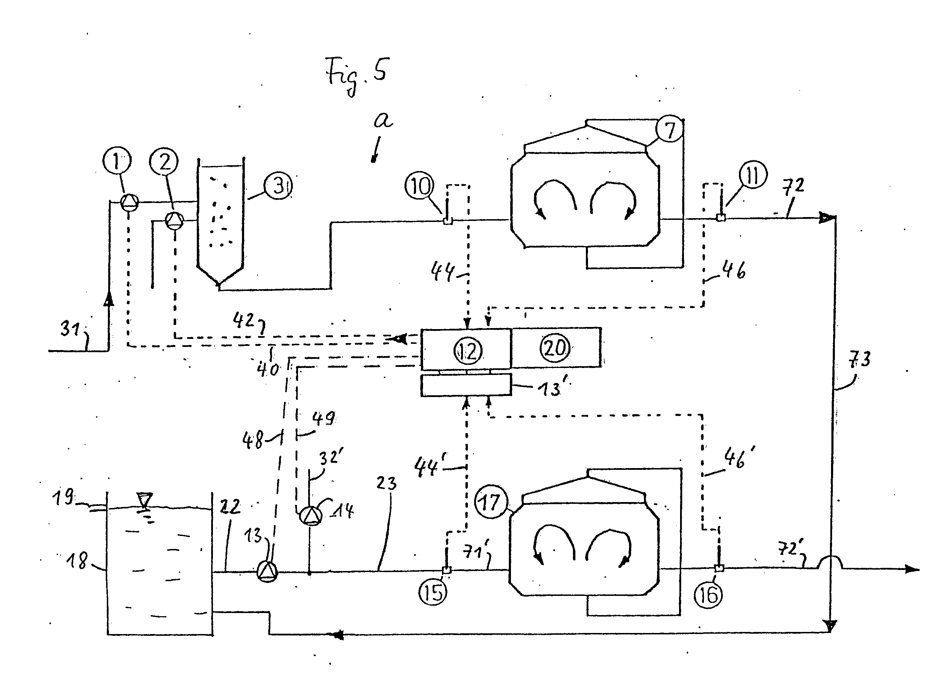

Die Einrichtung nach Fig. 5 kann auch zur Messung des Toxizitätsgrades benutzt werden, wenn die Leitung 73 in einen Tank 18 mündet, an den eine Entnahmeleitung 22 angeschlossen ist. Die Entnahmeleitung 22 führt über die Dosierpumpe 13 zur Sauerstoffelektrode 15 und von dort über die Zulaufleitung 71' zum zweiten Reaktionsbehälter 17. Der Tank 18 besitzt einen Überlauf 19. In den Leitungsabschnitt 23 zwischen Dosierpumpe 13 und Sauerstoffelektrode 15 mündet die Abstromseite der Dosierpumpe 14 für Verdünnungswasser aus der Zuleitung 32', wobei in den Leitungen Mittel vorgesehen sind, die Rückströmen von Flüssigkeit durch die Dosierpumpen 13, 14 verhindern. Gegebenenfalls muß in den Leitungsabschnitt 23 ein weiteres, nicht dargestelltes Belüftungsgefäß eingeschaltet werden. Die Steuerung ist dabei so eingestellt, daß die Dosierpumpen 1 und 2 von den Signalen aus den Sauerstoffelektroden 15 und 16 gesteuert werden. Die Dosierpumpe 13 entnimmt unter Steuerung über Leitung 48 dem Tank 18 um einen konstanten Faktor weniger Flüssigkeit als die Dosierpumpe 1 fördert. Die Dosierpumpe 14 wird über Leitung 49 so gesteuert, daß sie durch entsprechende Zugabe von Verdünnungswasser die erforderliche Volumenkonstanz der Strömung durch den Reaktionsbehälter 17 sicherstellt. Die Menge an Flüssigkeit, die über den Überlauf 19 am Tank 18 verdrängt wird, muß über die Pumpe 14 entsprechend den obengenannten Bedingungen zudosiert werden.

Wenn der die Leitung 73 durchfließende Teilstrom a um einen konstanten Faktor verdünnt wird, nimmt die Toxizität des über die Zulaufleitung 71' in den zweiten Reaktionsbehälter 17 eintretenden Teilstroms entsprechend der Verdünnung ab, so daß die Aktivität der Mikroorganismen im zweiten Reaktionsbehälter 17 nicht oder weniger gehemmt wird. Dies hat zur Folge, daß die Differenz der von den Sauerstoffelektroden 15 und 16 abgegebenen Meßwerte sich von der Differenz der von den Sauerstoffelektroden 10 und 11 gemessenen Konzentrationen unterscheidet. Aus diesem Unterschied der Differenzen ermittelt der Rechner 12 eine vorliegende Toxizität der Fracht im Teilstrom a. Die Pumpen 13 und 14 mischen den Flüssigkeitsstrom so, daß die Konzentration im Reaktionsbehälter 17 um einen konstanten Faktor geringer eingestellt ist als im Reaktionsbehälter 7, wobei die Volumenströme in beiden Behältern konstant sein müssen und gleich sein können.

Die Erfindung ist selbstverständlich auf Einzelheiten der vorstehend beschriebenen Ausführungsformen nicht beschränkt. So kann etwa bei der Einrichtung gemäß Fig. 4 die Sauerstoffelektrode 15 entfallen, wobei dann die Differenz der gemessenen Sauerstoffwerte zwischen den Elektroden 11 und 16 vom Rechner 12 verarbeitet wird. Auch kann dem zweiten Reaktionsbehälter 17 gegebenenfalls ein weiteres Belüftungsgefäß vorgeschaltet sein.

Vorbemessung:

| | |
|---|---|
| Reaktionszeit: | 5 Minuten |
| Volumenstrom: | 1 l/Min. |

Daraus folgt: Bei 20° C und 760 Torr (1 Torr = 133,3 Pa) stehen etwa 9 mg $O_2$/l, das sind 9 mg $O_2$/Min., zur Verfügung. Bei höherem Betriebsdruck läßt sich der zur Verfügung stehende Sauerstoff nach dem HENRYschen Gesetz $C_s = K_s \cdot P_t$ berechnen.

Der minimale Sauerstoffgehalt an der Elektrode 11 wird mit 2 mg $O_2$/l festgelegt. Der zulässige Sauerstoffverbrauch beträgt dann max. 7 mg/l.

Nach Voruntersuchungen entsteht bei einem spezifischen konstanten Nährstoffangebot $L_{k,a}$ (entsprechend 5 mg $BSB_5$/l) ein Sauerstoffbedarf von 2,5 g $O_2$/m²·d = 1,7 mg $O_2$/m²·Min. und bei $L_{k,b}$ (entsprechend 25 mg $BSB_5$/l) ein Sauerstoffbedarf von 5 g $O_2$/m²·d = 3,4 mg $O_2$/m²·Min.

Unter diesen Voraussetzungen wird ein Sauerstoffangebot von 7 mg/Min. durch den Einsatz von 4,1 bzw. 2,05 m² Aufwuchsfläche auf den Körpern 9 aufgezehrt.

Für die Körper werden gewählt:
Hohlzylinder, Innendurchmesser 3 mm, Höhe 3 mm, mit einer spezifischen Oberfläche von 2,1 m²/l bei lockerster Packung. Dies sind 37 037 Stück/l.

Der Bemessung entsprechend wird der Reaktionsbehälter 7 mit 72 310 Hohlzylindern (4,1 m²) und der Reaktionsbehälter 17 mit 36 155 Hohlzylindern (2,05 m²) gefüllt.

Die Hohlzylinder beanspruchen bei lockerster Lagerung 39 bzw. 19,5% des Reaktionsvolumens. Es besteht für sie somit noch ausreichend Bewegungsmöglichkeit.

Entsprechend der Vorbemessung ist $L_{k,a}$ mit 5 mg $BSB_5$/l und $L_{k,b}$ mit 25 mg $BSB_5$/l festgelegt.

Bei einem Mischungsverhältnis 1 : n wird das Gesamtnährstoffangeobt L — gemessen als $BSB_5$/l — wie folgt berechnet:

$$BSB_a = L_{k,a} \cdot (n+1) = L \ (mg \ BSB_5/l)$$

$$BSB_b = L_{k,b} \cdot \frac{(n+1)}{m} \cdot \frac{\Delta O_{2b}}{\Delta O_{2b,T}} \ (mg \ BSB_5/l).$$

Der Rechner verarbeitet den Wert $L_k$ nicht als Konstante, sondern erhöht oder erniedrigt je 0,1 mg $O_2$/l Abweichung vom Sollwert $\Delta O_2$ zwischen 10 und 11 die Werte $L_{k,a}$ und $L_{k,b}$ um 1,4%.

In der Rechnung bedeuten:

| | | |
|---|---|---|
| L | = | Nährstoffkonzentration |
| BSB | = | Biologischer Sauerstoffbedarf |
| $BSB_5$ | = | Biologischer Sauerstoffbedarf in 5 Tagen |
| $L_k$ | = | Konstante Nährstoffzufuhr gemessen als $BSB_5$ (mg/l) |
| a,b | = | Indizes für die Reaktionsverläufe in den Behältern 7 und 17 |
| $\Delta O_2$ | = | Eichwert, der aufgrund der zu erwartenden Abwasserschwankungen (Kläranlagenzulauf oder -ablauf) durch Variation der Aufwuchsflächen eingestellt wird |
| $\Delta O_{2b,T}$ | = | Kontrollwert, der mit dem Eichwert $\Delta O_{2b}$ verglichen wird |
| m | = | Faktor für die Vervielfachung der Nährstoffkonzentration im Behälter 17 |
| n | = | Verdünnungswasseranteil |
| $L_{(t)}$ | = | Nährstoffkonzentration zum Zeitpunkt »t« |

Funktionsbeschreibung der Meßeinrichtung (Fig. 1) anhand eines Rechenbeispiels, beginnend mit der Beschreibung eines stationären Analysezustandes (Verdünnungsverhältnis, Abwasser/Verdünnungswasser 1 : $n_1$).

Den Organismen im Reaktionsbehälter 7 wird eine konstante Nährstoffmenge $L_{k,a}$ (im Beispiel 5 mg $BSB_5$/l) zugeleitet. Das Mischungsverhältnis beträgt 1 : $n_1$ und der $BSB_a = L_{k,a} \cdot (n_1 + 1) = L$. Die Sauerstoffdifferenz zwischen den Elektroden 10 und 11 beträgt $\Delta O_{2a}$ (im Beispiel 7 mg $O_2$/l).

Bei Erhöhung der Schmutzkonzentration L reagieren die Organismen mit erhöhter Atmungsaktivität. Dadurch registrieren die Elektroden 10 und 11 eine größere Sauerstoffdifferenz. Bei $\Delta O_{2a} = 7,1$ mg $O_2$/l werden die Pumpen derart gesteuert, daß der Schmutzwasseranteil (Pumpe 1) verringert und der Verdünnungswasseranteil (Pumpe 2) erhöht wird, bis sich die $O_2$-Differenz zwischen 10 und 11 wieder auf 7 mg $O_2$/l eingestellt hat.

Das neue Mischungsverhältnis beträgt dann 1 : $n_2$ und der neue $BSB_a = L_{k,a} \cdot (n_2 + 1) = L$.

Bei Verringerung der Schmutzkonzentration L läuft der Prozeß in entgegengesetzter Richtung.

Bei parallelem Betrieb zweier Reaktionsbehälter (Fig. 2) zur BSB-Messung bei gleichzeitiger Toxizitätsüberwachung werden die Pumpen 1, 13 und 2, 14 in der zuvor beschriebenen Weise über die Elektroden 10 und 11 gesteuert. Die Pumpe 13 fördert aber das m-fache der Pumpe 1.

Die Elektroden 15 und 16 überwachen nur den Sauerstoffverbrauch im Reaktionsbehälter 17. Ent-

7

**0 049 887**

spricht die $O_2$-Differenz $\Delta O_{2b,T}$ dem Vorbemessungswert $\Delta O_{2b}$ (im Beispiel 7 mg $O_2$/l), liegt keine Hemmung vor. In diesem Fall sind $BSB_a$ und $BSB_b$ gleich.

Sind Toxizitätseinflüsse vorhanden, so gibt das Verhältnis $BSB_a$/$BSB_b$ in Verbindung mit dem Verdünnungsgrad 1 : n Aufschluß über den Grad der vorliegenden Zehrungshemmung.


**Patentansprüche**

1. Verfahren zur Messung des Verschmutzungsgrades von wäßrigen Lösungen, z. B. Abwasser, durch biologisch abbaubare Inhaltsstoffe, bei dem der zu untersuchenden Lösung ein Teilstrom entnommen wird, der mit Sauerstoff durchlüftet und kontinuierlich durch ein biologisches Bad in einem Reaktionsbehälter hindurchgeleitet wird, hinter dem sein Restsauerstoffgehalt gemessen wird, wobei die Lebendmasse des Bades im wesentlichen auf konstantem Niveau gehalten wird, dadurch gekennzeichnet, daß der Teilstrom mit biologisch neutralem Wasser verdünnt wird und daß die Verdünnung des Teilstroms durch Messung des Sauerstoffgehaltes vor dem Reaktionsbehälter und Vergleich mit dem Meßergebnis des Restsauerstoffgehaltes derart geregelt wird, daß bei konstantem Volumenstrom durch den Reaktionsbehälter die Differenz der Meßwerte im wesentlichen bei einem vorgegebenen Wert konstant bleibt, wobei der Verdünnungsgrad zur Anzeige des Verschmutzungsgrades dient.

2. Verfahren nach Anspruch 1, dadurch gekennzeichnet, daß die Konzentration des dem Reaktionsbehälter zugeführten verdünnten Nährstoffstroms aus dem Bereich der angenähert linearen Abhängigkeit der Reaktionsgeschwindigkeit der Lebendmasse vom Nährstoffangebot gewählt wird.

3. Verfahren nach Anspruch 1 oder 2, dadurch gekennzeichnet, daß die Konzentration des dem Reaktionsbehälter zugeführten verdünnten Nährstoffstroms so gewählt wird, daß die Reaktionsgeschwindigkeit der Lebendmasse die halbe maximale Reaktionsgeschwindigkeit nicht übersteigt.

4. Verfahren nach einem der vorstehenden Ansprüche, dadurch gekennzeichnet, daß das konstante Nährstoffangebot, gemessen als biologischer Sauerstoffgehalt in 5 Tagen, zwischen 1 und 15 mg/l liegt.

5. Verfahren nach einem der vorstehenden Ansprüche, dadurch gekennzeichnet, daß der zu untersuchenden Flüssigkeit gegen hemmende Wirkungen von Schwankungen des pH-Wertes eine biologisch neutrale Pufferlösung zugesetzt wird.

6. Verfahren nach den vorhergehenden Ansprüchen, dadurch gekennzeichnet, daß es unter leichtem Überdruck abläuft.

7. Verfahren nach einem der vorstehenden Ansprüche, dadurch gekennzeichnet, daß der zu untersuchenden Lösung ein zweiter Teilstrom entnommen und in gleicher Weise behandelt wird, wobei jedoch der Verdünnungsgrad des zweiten Teilstroms um einen konstanten Faktor m niedriger eingeregelt wird als der des ersten Teilstroms und die Veränderung der Differenz der Meßwerte vor und hinter dem zweiten Reaktionsbehälter mit der Veränderung der Differenz der Meßwerte aus dem ersten Teilstrom verglichen und als Toxizitätsgrad angezeigt wird.

8. Verfahren nach Anspruch 7, dadurch gekennzeichnet, daß der Faktor m nach der Art der erwarteten Toxizität zwischen 2 und 20 gewählt wird.

9. Verfahren nach einem der vorstehenden Ansprüche, dadurch gekennzeichnet, daß in dem biologischen Bad eine Vielzahl freier Körper mit Aufwuchsflächen für die Lebendmasse in ständiger turbulenter Bewegung gehalten wird.

10. Einrichtung zur Ausführung des Verfahrens nach einem der vorstehenden Ansprüche mit einem Belüftungsgefäß (3) und einem Reaktionsbehälter (7), durch die der aus der wäßrigen Lösung entnommene Teilstrom hindurchfließt, mit einer Sauerstoffelektrode (11) in der Ablaufleitung (72) aus dem Reaktionsbehälter sowie einer weiteren Sauerstoffelektrode (10) in einer den Auslauf (33) des Belüftungsgefäßes (3) mit dem Zulauf (71) des Reaktionsbehälters (7) verbindenden Leitung (60), dadurch gekennzeichnet, daß auf der Einlaufseite des Belüftungsgefäßes (3) die Zuleitung (31) des Teilstroms mit einer Zuleitung (32) für Verdünnungswasser zusammengeführt ist, daß in der Zuleitung (32) für Verdünnungswasser eine an eine Steuerung (12) angeschlossene Durchfluß-Regelvorrichtung (2) liegt und daß die weitere Sauerstoffelektrode (10) an die Steuerung (12) angeschlossen ist, wobei die Steuerung einen Vergleicher und einen Stellsignalgeber für die Durchfluß-Regelvorrichtung (2) enthält.

11. Einrichtung nach Anspruch 10, dadurch gekennzeichnet, daß in der Zuleitung (31) für den Teilstrom und in der Zuleitung (32) für Verdünnungswasser je eine Dosierpumpe (1, 2) angeordnet sind.

12. Einrichtung nach einem der Ansprüche 10 oder 11, dadurch gekennzeichnet, daß der Reaktionsbehälter (7) in eine Umwälz-Leitung (81) geschaltet ist, durch die mittels einer weiteren Pumpe (8) der flüssige Inhalt des Reaktionsbehälters (7) kontinuierlich umgepumpt wird.

13. Einrichtung nach einem der Ansprüche 10 bis 12, dadurch gekenzeichnet, daß in dem im Reaktionsbehälter (7) enthaltenen biologischen Bad eine Vielzahl freier Körper (9) mit Aufwuchsflächen für die Lebendmasse enthalten ist.

14. Einrichtung nach Anspruch 13, dadurch gekennzeichnet, daß die freien Körper (9) gegen mechanische Beeinflussung geschützte Aufwuchsflächen besitzen.

8

15. Einrichtung nach Anspruch 13 oder 14, dadurch gekennzeichnet, daß die freien Körper (9) offene Hohlkörper sind, die im Inneren freie Aufwuchsflächen besitzen.

16. Verfahren nach einem der Ansprüche 1 bis 6 oder 9, dadurch gekennzeichnet, daß der den Reaktionsbehälter (7) verlassende Teilstrom durch einen ein biologisches Bad enthaltenden zweiten Reaktionsbehälter (17) geführt und die Differenz des Sauerstoffverbrauchs m ersten Reaktionsbehälter und des Sauerstoffverbrauchs im zweiten Reaktionsbehälter als Maß für die Abbaubarkeit der Verschmutzung des Teilstroms ermittelt wird.

17. Verfahren nach einem der Ansprüche 1 bis 6 oder 9, dadurch gekennzeichnet, daß der den Reaktionsbehälter verlassende Teilstrom nach Verdünnung durch einen zweiten Reaktionsbehälter (17) geführt wird und daß die Verdünnung des in den ersten Reaktionsbehälter eintretenden Teilstroms derart geregelt wird, daß bei konstantem Volumenstrom durch den zweiten Reaktionsbehälter die Differenz der Meßwerte aus dem zweiten Reaktionsbehälter im wesentlichen nach einem vorgegebenen Wert konstant bleibt, wobei der Verdünnungsgrad des Teilstroms im zweiten Reaktionsbehälter um einen konstanten Faktor m höher eingeregelt wird als der des ersten Reaktionsbehälters und die Veränderung der Differenz der Meßwerte vor und hinter dem zweiten Reaktionsbehälter mit der Veränderung der Differenz der Meßwerte aus dem ersten Teilstrom verglichen und als Toxizitätsgrad angezeigt wird.

18. Einrichtung nach einem der Ansprüche 10 bis 15 zur Ausführung des Verfahrens nach Anspruch 16, dadurch gekennzeichnet, daß die Auslaufleitung (72) des ersten Reaktionsbehälters (7) mit der Einlaufleitung (71') eines zweiten Reaktionsbehälters (17) verbunden ist, wobei dem zweiten Reaktionsbehälter (17) eine weitere Sauerstoffelektrode (16) nachgeschaltet ist und über eine Signalleitung (46') mit der Steuerung (12, 13) verbunden ist.

19. Einrichtung nach Anspruch 18 zur Ausführung des Verfahrens nach Anspruch 17, dadurch gekennzeichnet, daß die Auslaufleitung (72) in einen mit Überlauf (19) ausgerüsteten Tank (18) mündet, an den eine Entnahmeleitung (22) mit Dosierpumpe (13) angeschlossen ist, deren Abstromseite mit der Zulaufleitung (71') des zweiten Reaktionsgefäßes (17) gekoppelt ist, wobei eine Verdünnungswasserzuleitung (32') mit Dosierpumpe (14) mit der Zulaufleitung (71') gekoppelt ist und je eine Sauerstoffelektrode (15, 16) dem zweiten Reaktionsgefäß (17) vor- und nachgeschaltet ist.

20. Einrichtung nach einem der Ansprüche 10 bis 15 zur Ausführung des Verfahrens nach einem der Ansprüche 7 bis 9, dadurch gekennzeichnet, daß der Einrichtung eine weitere gleichartige Einrichtung (b) mit einem zweiten Belüftungsgefäß (3'), einem zweiten Reaktionsbehälter (17) und je einer vor und hinter dem zweiten Reaktionsbehälter (17) angeordneten Sauerstoffelektrode (15, 16) parallel geschaltet ist, wobei eine dem zweiten Belüftungsgefäß (3') vorgeschaltete Durchfluß-Regelvorrichtung (13, 14) von der Steuerung (12) derart beaufschlagt ist, daß der Verdünnungsgrad des zweiten Teilstroms sich um einen konstanten Faktor m vom Verdünnungsgrad des ersten Teilstroms unterscheidet.

## Claims

1. A process for measuring the degree of pollution of aqueous solutions, for example, effluent, in the form of biodegradable components, in which a partial stream is withdrawn from the liquid undergoing examination is oxygenated and caused to flow continuously through a biological bath in a reaction vessel after which its residual oxygen content is measured, in the course of which the living mass of the bath is held at an essentially constant level, characterized in that the partial stream is diluted with biologically neutral water and that the dilution of the partial stream is regulated in such a manner through measurement of the oxygen content ahead of the reaction tank and comparison with the result of measuring the residual oxygen that with constant volume flow through the reaction vessel, the difference between the values obtained remains essentially constant at a predetermined value, so that the level of dilution serves for the indication of the level of pollution.

2. The process according to claim 1 characterized in that the concentration of the diluted nutrient stream supplied to the reaction vessel is selected from the region of the approximately linear slope of the dependency of the reaction speed of the living mass to the nutrient supply.

3. The process according to claim 1 or 2 characterized in that the concentration of the diluted nutrient stream supplied to the reaction vessel is so selected that the reaction speed of the living mass does not exceed half the maximum reaction speed.

4. A process according to one of the foregoing claims characterized in that the constant nutrient supply, measured as biological oxygen demand in 5 days, is between 1 and 15 mg/l.

5. The process according to one of the foregoing claims characterized in that the liquid undergoing examination is mixed with a biologically neutral buffer solution against the inhibiting action of variations in the pH value.

6. The process according to one of the foregoing claims characterized in that it is carried out under slightly elevated pressure.

7. The process according to one of the foregoing claims characterized in that a second partial stream is withdrawn from the liquid undergoing examination and is treated in a similar manner, in the course of which however the level of dilution of the second partial stream is adjusted lower by a constant

9

factor (m) than that of the first partial stream and the change of the difference of the measured values before and after the second reaction vessel is compared with the change of the difference of the measured values from the first partial stream and is indicated as level of toxicity.

8. The process according to claim 7 characterized in that the factor (m) is selected between 2 and 20 in accordance with the type of the expected toxicity.

9. The process according to one of the foregoing claims characterized in that in the biological bath a plurality of free bodies with growth surfaces for the living mass are maintained in constantly turbulent movement.

10. Apparatus for carrying out the process according to one of the foregoing claims with an aeration tank (3) and a reaction vessel (7) through which the partial stream withdrawn out of the aqueous solution flows, and with an oxygen electrode (11) in the discharge conduit of the reaction vessel, characterized in that, on the inlet side of the aeration tank (3), the supply conduit (31) of the partial stream is brought together with a supply conduit (32) for the dilution water, that a flow regulating apparatus (2) connected to a control (12) lies in the supply conduit (32) for the dilution water and that a further oxygen electrode (10) also connected to the control (12) is arranged in a conduit (60) connecting the discharge (33) of the aeration tank (3) with the feed (71) of the reaction vessel (7), wherein the control includes a comparator and a regulating signal generator for the flow control device (2).

11. Apparatus according to claim 10 characterized in that in each of the supply conduit (31) for the partial stream and in the supply conduit (32) for the dilution water, a metering pump 1, 2, is arranged.

12. The apparatus according to one of the claims 10 or 11 characterized in that the reaction vessel (7) is connected in a recirculating conduit (81), through which the fluid contents of the vessel (7) are continuously circulated by means of a further pump (8).

13. The apparatus according to one of the claims 10 through 12 characterized in that, in the biological bath contained in the reaction vessel (7), a plurality of free bodies (9) with growth surfaces for the living mass is contained.

14. The apparatus according to claim 13 characterized in that the free bodies (9) possess growth surfaces protected against mechanical influences.

15. The apparatus according to claims 13 or 14 characterized in that the free bodies (9) are open hollow bodies that in the interior possess free growth surfaces.

16. The process according to one of the claims 1 through 6 or 9 characterized in that the partial stream leaving the reaction vessel (7) is fed through second reaction vessel (17) containing a biological bath and the difference of the oxygen consumption in the first reaction vessel and the oxygen consumption in the second reaction vessel is established as a measurement for the degradability of the pollution of the partial stream.

17. A process according to one of the claims 1 through 6 or 9 characterized in that the partial stream leaving the reaction vessel after dilution is fed through fa second reaction vessel (17) and that the dilution of the partial stream entering into the first reaction vessel is regulated in such a manner that with constant flow volume through the second reaction vessel the difference of the measured values from the second reaction vessel remains essentially constant at a predetermined value, wherein the dilution level of the partial stream in the second reaction vessel is regulated higher by a constant factor (m) than that of the first reaction vessel and the change of the difference of the measured values before and after the second reaction vessel is compared with the change of the difference of the measured values from the first partial stream and is indicated as the level of toxicity.

18. The apparatus according to one of the claims 10 through 15 for carrying out the process according to claim 16 characterized in that the discharge conduit (72) of the first reaction vessel (7) is connected with the inlet conduit (71') of the second reaction vessel (17) wherein a further oxygen electrode (16) is connected downstream of the second reaction vessel (17) and is connected through a signal conductor (46') with the control (12, 13).

19. The apparatus according to claim 18 for carrying out the process according to claim 17 characterized in that the discharge conduit (72) opens in a tank (18) equipped with an overflow (19) in which a drawing off conduit (22) with metering pump (13) is connected to the downstream side of which is coupled with the supply conduit (71') of the second reaction vessel (17), wherein a dilution water supply conduit (32') with metering pump (14) is connected to the supply conduit (71') and an oxygen electrode (15, 16) is connected ahead and behind the second reaction vessel (17).

20. Apparatus according to one of the claims 10 through 15 for carrying out the process according to one of claims 7 through 9 characterized in that the apparatus is connected in parallel with a further similar apparatus (b) with a second aeration tank (3'), a second reaction vessel (17) and an oxygen electrode (15, 16) arranged ahead and behind the second reaction vessel (17), wherein a flow control device (13, 14) connected ahead of the second aeration tank (3') is caused by the control (12) to act in such a manner that the dilution level of the second partial stream differs by a constant factor (m) from the dilution level of the first partial stream.

**0 049 887**

### Revendications

1. Procédé de mesure du degré de pollution des solutions aqueuses, part exemple des eaux usées, par des substances biodégradables, dans lequel une partie du flux de la solution à étudier est prélevé, oxygéné et dirigé en continu dans un bain biologique dans une cuve de réaction, en aval de laquelle la teneur résiduelle en oxygène est mesurée, la biomasse du bain étant essentiellement maintenue constante, caractérisé en ce que le flux partiel est dilué d'eau biologiquement neutre et en ce que la dilution du flux partiel est réglée sur la base d'une mesure de la teneur en oxygène en amont de la cuve de réaction et d'une comparaison avec le résultat de la mesure de la teneur résiduelle en oxygène de manière à ce que, pour un volume constant traversant le réacteur, la différence des valeurs de mesure reste essentiellement constnate et égale à une valeur prédéterminée, si bien que le degré de dilution est utilisé pour représenter le degré de pollution.

2. Procédé selon la revendication 1, caractérisé en ce que la concentration du flux de substance nutritive diluée apporté à la cuve de réaction est choisie dans le domaine de relation quasi-linéaire entre la vitesse de réaction de la biomasse et l'offre de nourriture.

3. Procédé selon la revendication 1 ou 2, caractérisé en ce que la concentration du flux de substance nutritive diluée apporté à la cuve de réaction est choisie de manière à ce que la vitesse de réaction de la biomasse ne dépasse pas la moitié de la vitesse de réaction maximale.

4. Procédé selon l'une des revendications précédentes, caractérisé en ce que l'offre constante de substances nutritives, mesurée sous la forme de demande biologique d'oxygène en 5 jours, est comprise entre 1 et 15 mg/l.

5. Procédé selon l'une des revendications précédentes, caractérisé en ce que le liquide à étudier est additionné d'une solution tampon biologiquement neutre pour éviter les actions inhibitrices des variations du pH.

6. Procédé selon les revendications précédentes, caractérisé en ce qu'il se déroule sous une légère surpression.

7. Procédé selon l'une des revendications précédentes, caractérisé en ce qu'un second flux partiel est prélevé dnas la solution à étudier et traité de la même manière, mais avec un degré de dilution du second flux partiel réduit d'un facteur constant m par rapport au premier flux partiel, et que la variation de la différence des valeurs de mesure en amont et en aval de la seconde cuve de réaction est comparée avec la variation des différences des valeurs de mesure sur le premier flux partiel et affichée comme degré de toxicité.

8. Procédé selon la revendication 7, caractérisé en ce que le facteur m est choisi entre 2 et 20 en fonction de la nature de la toxicité à attendre.

9. Procédé selon l'une des revendications précédentes, caractérisé en ce que l'on maintient en mouvement turbulent constant dans le bain biologique une multitude de corps libres portant des surfaces de croissance pour la biomasse.

10. Dispositif de mise en œuvre du procédé selon l'une des revendications précédentes, avec un réservoir d'aération (3) et une cuve de réaction (7) par lesquels passe le flux partiel prélevé dans la solution aqueuse, avec une électrode à oxygène (11) sur la conduite d'évacuation (72) sortant de la cuve de réaction (7) et avec une autre électrode à oxygène (10) sur une conduite (60) reliant la sortie (33) du réservoir d'aération (3) et l'entrée (71) de la cuve de réaction (7), caractérisé en ce que, sur le côté admission du réservoir d'aération (3), la conduite d'admission du flux partiel (31) est réunie à une conduite d'admission d'eau de dilution (32), en ce que, sur la conduite d'admission de l'eau de dilution (32), se trouve un dispositif de réglage du débit (2) raccordé à une commande (12) et en ce que l'autre électrode à oxygène (10) est raccordée à la commande (12), celle-ci comprenant un comparateur et un émetteur de signaux d'asservissement pour le dispositif de réglage du débit (2).

11. Dispositif selon la revendication 10, caractérisé en ce que la conduite d'admission (31) du flux partiel et la conduite d'admission (32) de l'eau de dilution sont dotées chacune d'une pompe doseuse (1, 2).

12. Dispositif selon l'une des revendications 10 ou 11, caractérisé en ce que la cuve de réaction (7) est montée sur une conduite de recirculation (81) par laquelle, grâce à une autre pompe (8) le contenu liquide de la cuve de réaction (7) est pompé en permanence.

13. Dispositif selon l'une des revendications 10 à 12, caractérisé en ce que de bain biologique contenu dans la cuve de réaction (7) contient une multitude de corps libres (9) portant des surfaces de croissance pour la biomasse.

14. Dispositif selon la revendication 13, caractérisé en ce que les corps libres (9) possèdent des surfaces de croissance protégées contre les influences mécaniques.

15. Dispositif selon la revendication 13 ou 14, caractérisé en ce que les corps libres (9) sont des corps creux ouverts possédant à l'intérieur des surfaces de croissance libres.

16. Procédé selon l'une des revendications 1 à 6 ou 9, caractérisé en ce que le flux partiel quittant la cuve de réaction (7) est conduit dans une seconde cuve de réaction (17) contenant un bain biologique et en ce que l'on détermine la différence entre la consommation d'oxygène dans la première cuve de réaction et dans la seconde cuve de réaction pour mesurer la dégradabilité de la pollution du flux partiel.

11

17. Procédé selon l'une des revendications 1 à 6 ou 9, caractérisé en ce que le flux partiel quittant la cuve de réaction est conduit après dilution dans une seconde cuve de réaction (17) et en ce que la dilution du flux partiel entrant dans la première cuve de réaction est réglée de manière à ce que, pour un débit volumique constant dans la seconde cuve de réaction, la différence des valeurs mesurées sur la seconde cuve de réaction reste essentiellement constante et égale à une valeur prédéterminée, le degré de dilution du flux partiel dans la seconde cuve de réaction étant supérieur d'un facteur constant m à celui utilisé pour la première cuve de réaction et la variation des différences des valeurs mesurées en amont et en aval de la seconde cuve de réaction étant comparée avec la variation de la différence des valeurs mesurées sur le premier flux partiel et affichée comme degré de toxicité.

18. Dispositif selon l'une des revendications 10 à 15 pour la mise en œuvre du procédé selon la revendication 16, caractérisé en ce que la conduite d'évacuation (72) de la première acuve de réaction (7) est reliée à la conduite d'admission (71') d'une seconde cuve de réaction (17), une autre électrode à oxygène (16) étant montée en aval de la seconde cuve de réaction (17) et reliée par une ligne de transmission de signaux (46') avec la commande (12, 13).

19. Dispositif selon la revendication 18 pour la mise en œuvre du procédé selon la revendication 17, caractérisé en ce que la conduite d'évacuation (72) débouche dans une cuve (18) dotée d'un trop-plein (19) sur laquelle est raccordée une conduite de prélèvement (22) avec une pompe doseuse (13) dont le côté refoulement est raccordé à la conduite d'admission (71') de la seconde cuve de réaction (17), la conduite d'eau de dilution (32') avec pompe doseuse (14) étant couplée à la conduite d'admission (71') et une électrode à oxygène (15, 16) étant montée en amont et en aval de la seconde cuve de réaction (17).

20. Dispositif selon l'une des revendications 10 à 15 pour la mise en œuvre du procédé selon l'une des revendications 7 à 9, caractérisé en ce que le dispositif est monté en parallèle à un second dispositif similaire (b) ayant un second réservoir d'aération (3'), une seconde cuve de réaction (17) et une électrode à oxygène (15, 16) montée en amont et en aval de la seconde cuve à réaction (17), un dispositif de réglage de débit (13, 14) relié en amont du second réservoir d'aération (3') étant piloté par la commande (12) de sorte que le degré de dilution du second flux partiel se différencie du degré de dilution du premier flux partiel d'un facteur constant m.

Fig 1

Fig. 2

Fig. 3

$$V = V_{max} \cdot \frac{L}{k_m + L}$$

$$n+1 = \frac{L}{L_k}$$

Reaktionsgeschwindigkeit

Verdünnungsverhältnis 1:n

Nährstoffangebot

Fig. 4

Fig. 5